Europäisches Patentamt

**European Patent Office**

**Office européen des brevets**

(11) Publication number: **0 158 298**
**A2**

(12) **EUROPEAN PATENT APPLICATION**

(21) Application number: **85104161.6**

(22) Date of filing: **04.04.85**

(51) Int. Cl.⁴: **A 61 B 1/00**
**A 61 B 5/00, G 01 N 21/27**

(30) Priority: **05.04.84 JP 67878/84**

(43) Date of publication of application:
**16.10.85 Bulletin 85/42**

(84) Designated Contracting States:
**DE FR GB IT**

(71) Applicant: **SUMITOMO ELECTRIC INDUSTRIES LIMITED**
**No. 15, Kitahama 5-chome Higashi-ku**
**Osaka-shi Osaka-fu(JP)**

(72) Inventor: **Hiramoto, Junichi c/o Sumitomo Electric Ind.**
**Ltd.**
**Osaka Works 1-3, Shimaya 1-chome**
**Konohana-ku, Osaka(JP)**

(72) Inventor: **Yotsuya, Koro c/o Sumitomo Electric Ind. Ltd.**
**Osaka Works 1-3 Shimaya 1-chome**
**Konohana-ku Osaka(JP)**

(74) Representative: **KUHNEN & WACKER**
**Patentanwaltsbüro**
**Schneggstrasse 3-5 Postfach 1729**
**D-8050 Freising(DE)**

(54) **A probe for abnormality of internal tissue by spectroscopic method.**

(57) An improved probe for measuring abnormality of living human tissue is disclosed. The probe (23) is equipped with means (21, 22) for detecting the degree of contact of its measuring end portion (6) with the surface of an object of human tissue (13) and is capable of being applied through opening space for forceps in endoscops.

FIG. 7

Patentanwälte/European Patent Attorneys:
Rainer A. Kuhnen*, Dipl.-Ing.
Paul-A. Wacker*, Dipl.-Ing., Dipl.-Wirtsch.-Ing.
Wolfgang Luderschmidt**, Dr., Dipl.-Chem.

Sumitomo Electric

Industries, Ltd.

Osaka, Japan

56 SUO5 13 3/ze

# A PROBE FOR ABNORMALITY OF INTERNAL TISSUE BY SPECTROSCOPIC METHOD

## Field of the Invention

The present invention relates to a probe for detecting abnormality of internal tissue utilizing fiber optic means, and more particularly, to a probe for a spectrum analysis of reflected light by living tissue or organ of human which are kept in touch with it. The probe of the present invention can be inserted within human body through an opening of endoscopic device without difficulty.

## Prior Art

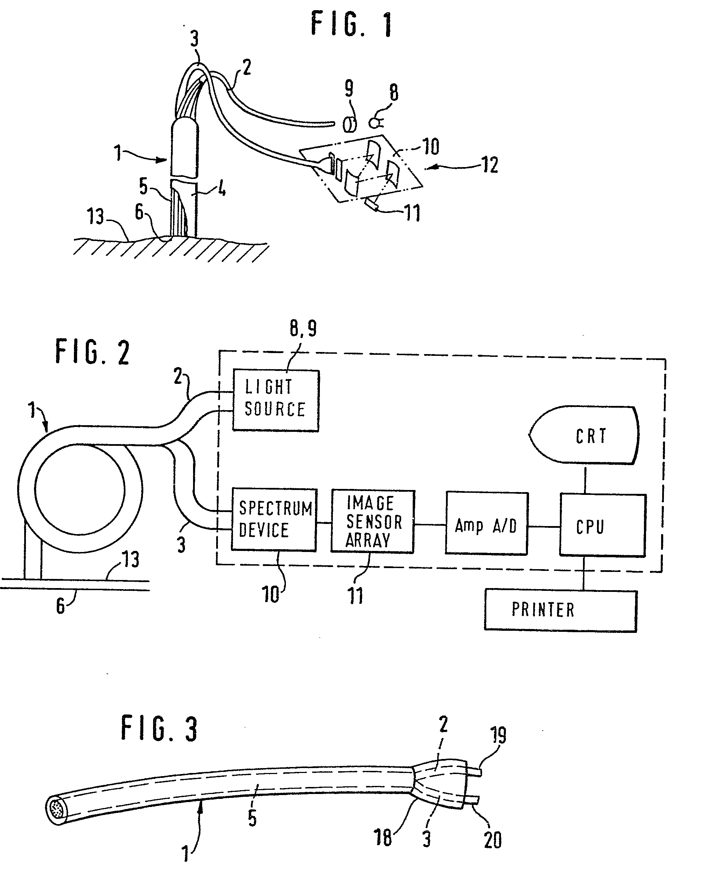

The method and its principle for measuring absorbance of light which is projected on human tissue or more practically those for spectrum-analysis of the reflected beam from the tissue to be observed are shown in Fig. 1. the corresponding data-processing flow is shown in Fig. 2.

**Büro Frankfurt/Frankfurt Office:**

Adenauerallee 16    Tel. 06171/300-1
D-6370 Oberursel    Telex: 526547 pawa d

*Büro München/Munich Office:

Schneggstraße 3-5    Tel. 08161/6209-1
D-8050 Freising      Telex 526547 pawa d

Telegrammadresse: Pawamuc — Postscheck München 136052-802
Telefax: 08161/6209-6 (GP. 2+3) — Teletex 8161800=pawaMUC

In Fig. 1 and Fig. 2, a probe 1 consists of optical fiber light-guide 2 and 3, in which the former is for projecting light beam on to surface of an tissue and the latter is for receiving the reflected beams therefrom. As shown in Fig. 1 the two optical wave-guides are combined together to form a unified bundle 5 of optical fibers within a protective sheath 4 in their one end portion whose cross-sectional (radial cross-section) portion 6 faces the object tissue.

The other end portion of the two groups of optical fibers 2 and 3 are kept separated and a light source 8, focusing lens 9 are connected to one of the bundles 2, while the remaining light-receiving optical fibers 3 are connected to a spectral apparatus 12 containing a spectrum-diffraction device 10, and an image sensor array 11.

The circular end portion 6 faces the object tissue and its surface is illuminated by beams from light source 8 through focussing lens 9 and optical waveguide 2. The reflective light beams are transmitted to the spectral apparatus 12 where their spectroscopic analysis is carried out. The results of the analysis are displayed either on a cathode ray tube (CRT) or printed by a graphic printer.

Fig. 3 illustrates configuration of such a probe for practical diagnostic use. A connector portion is formed where the branching portion of the bundle into two fiber waveguides 2 and 3 are included. To each of the divided waveguides 2 and 3 metal connecting plug 19 and 20 are attached. The probe 1 in the figure is connected to light source 8 and to spectral apparatus 12 by means of connecting portion 18.

In order to achieve an accurate measurement by utilizing such a probe as described above, it is necessitated that the annular contact surface of the probe is in an appropriate state of contact with the object tissue portion.

A method of observing a good contact of the sensing surface of the probe with the object tissue surface is shown in Fig. 4. An electrode 15 is attached to the object human body 14 for grounding purpose and weak electric current with high frequency is applied between the electrode 15 and a sleeve 16 which is attached to an end portion of the probe. The value of impedance measured when a sleeve 16 is in contact with human body 14 is taken as an index of the degree of contact between them. A circuit for detecting such an index is illustrated in Fig. 5 and some configurational view of the sleeve 16 and a lead-wire 17 shown in Fig. 6.

Problems arise from the fact that the measuring probe for spectrum analysis of human tissue is usually applied together with an endoscope having some small opening called "a forceps window" through which the probe is lead into human body until it reaches to the surface of the object tissue. As the sleeve 16 is placed around the outer circumference of an end portion of the probe, the radius of that end portion will become larger than usual. For instance in the case of gastroscopic analysis, the surface of the object tissue is gastro-mucous membrane and it causes difficulties for phycician to apply a probe with such an outside sleeve into stomach through an opening originally designed for inserting forceps.

These difficulties are further increased by the fact that the lead wire 17 is wired outwardly along the probe separately which also leads to enlargement of the probe and diminishes its operability.

4

The problem underlying the invention is therefore to provide a probe capable of detecting the degree of contact with an object human tissue and of being introduced through openings for forceps in an endoscopic configuration into human body without difficulty.

The solution of this problem is achieved by the characterizing features of claims 1 and 2.

## Summary of the Invention

According to the present invention a probe is provided comprising a metal ring which has about the same outer diameter as the fiber bundle with sheath. Said metal ring is placed around the circumferencial surface of the measuring end portion of the probe and is electrically connected with either one of metal tips attached to optical fiber wave guides for light projection or the ones for receiving reflected light beams from the object tissue.

## Brief Description of the Drawings

The invention will now be described by way of example, with reference to the accompanying drawings in which:

Fig. 1 shows a whole scope of the spectro-analyzer for human tissue utilizing an optical probe in general.

Fig. 2 shows total functional featurs of the spectro-analyzer utilizing an optical probe in general.

Fig. 3 illustrates a schematic view of an optical probe in general.

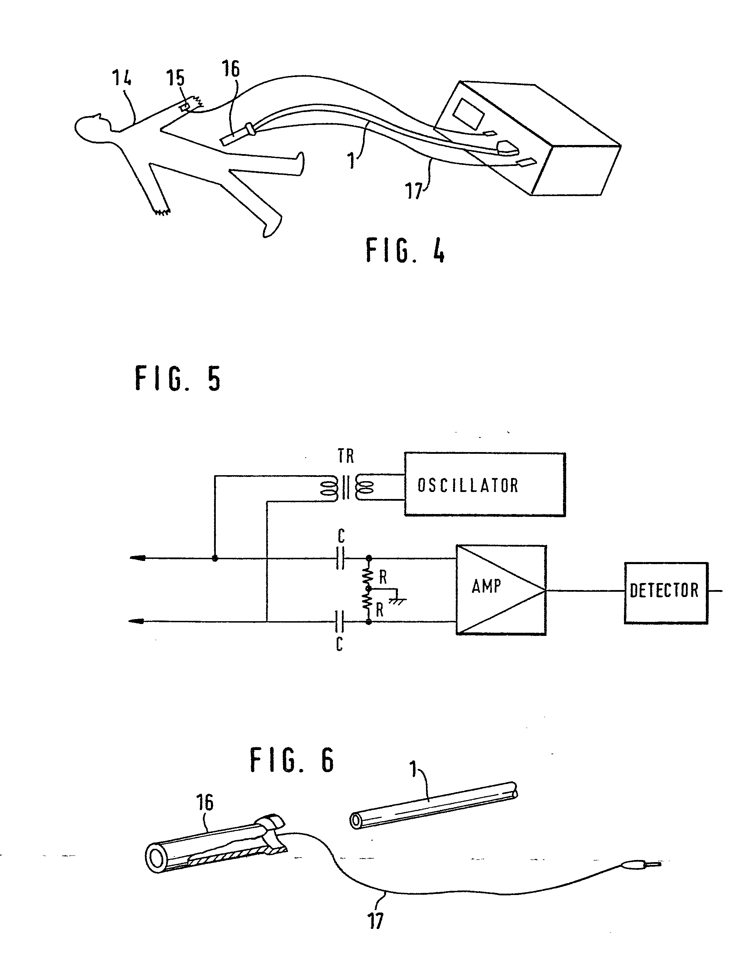

Fig. 4 shows a method of detecting degree of contact of an optical probe with human tissue.

Fig. 5 shows circuit for measuring contact impedance as a measure of the degree of contact of an optical probe with human tissue.

Fig. 6 shows constraction of conventional type of optical probe which carries lead wire separately.

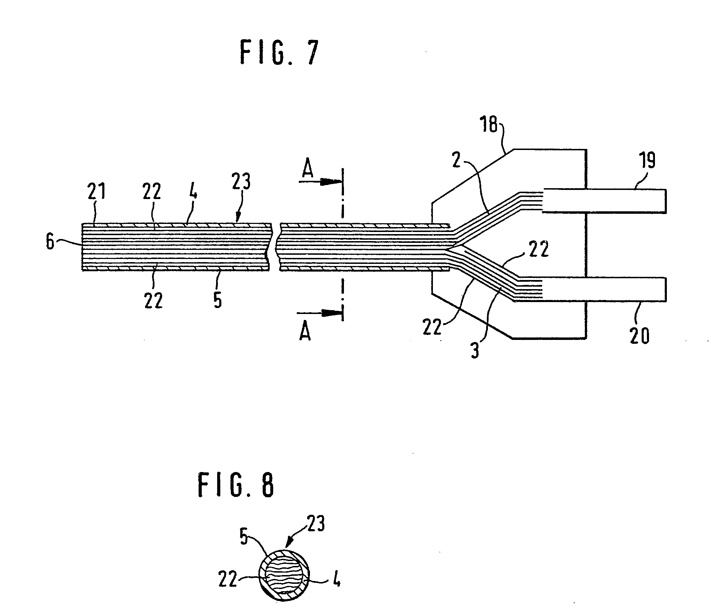

Fig. 7 shows a longitudinal cross-section of an embodiment of the probe of the present invention.

Fig. 8 shows a cross-sectional view of the probe illustrated in Fig. 7 along line A-A.

Description of the Preferred Embodiments

Fig. 7 shows a longitudinal cross-section of the probe of the present invention and Fig. 8 shows also a cross-sectional view of the probe of the present invention which is taken at the plane corresponding to A-A in Fig. 7. The probe 23 has basicly similar configuration to the one shown in Fig. 3.

Optical fiber waveguide for sending projection beams 2 and those for receiving reflected beams 3 are bundled together within a covering layer to form one bundle of optical fibers 5 in a common protective sheath 4 and its one end portion becomes the measuring end portion 6.

The bundled fibers are divided again at the connecting portion 18 into former two waveguide parts of 2 and 3 and metal tips 19 and 20 are connected to each of them respectively. In the probe of the present invention, the measuring end portion 6 of the probe has a metal ring 21 at its end portion of almost equal outer diameter as that of the fiber bundle 5 with sheath layer instead of protective sheath 4.

The inside circumferential surface of the metal ring 21 directly contacts with the outer circumference of the fiber bundle 5 in its longitudinal direction and shares one common plane 6 in its radial direction, so that the metal ring 21 has a core portion which is composed of optical fibers as shown in Fig. 8.

The outer surface of the metal ring 21 has been shaped so that its outer diameter diminishes stepwisely, and is fixed under the protective sheath 4. As illustrated in Fig. 8, at least one metalic lead wire 22 is located in the fiber bundle 5 under the protective sheath 4 and is incorporated into either of the optical fiber waveguide for receiving reflected beams 3 or for sending projection beam. Then the metal ring 21 can be electrically connected to either of the metal connecting plug 19 or 20 by means of lead wire 22. The lead wire 22 can be designed so as to be enough fine to transmit very weak electrical signals while keeping the outer diameter of the fiber bundle unchanged.

By connecting the metal connecting plug 20 with terminal T in Fig. weak electric current of high frequency will be transmitted through lead wire 22 to the metal ring 21 which is placed around the circumferential surface of the measuring end portion 6 of the probe 23 and thus the electric current is transferred between electrode for grounding 15 and metal ring 21 when the measuring end portion 6 has become attached to the surface of the object tissue 13. The observed value of contact impedance provides an indication of the degree of contact between the probe and the object tissue.

As described above, the present invention provides an improved probe for detecting abnormality of living tissue by spectro-analytical principle which allows its handling with conventional endoscopes with opening for forceps and is capable of assuring steady contact of its measuring end portion to the object tissue.

Sumitomo Electric Industries, Ltd.          56 SU05 13 3/ze
Osaka,
Japan

## Claims

1. A probe for detecting abnormality of internal tissue by spectroscopic method having a bundle of optical fibers (5) whose one end is used as measuring end portion (6) and the other end portion is incorporated in a connecting portion (18) where the bundle is divided into two groups (2, 3), namely one for transmission of the projection light beams to an object tissue (13) and the other for receiving reflected light beams from the object tissue, characterized by comprising a connecting plug of metal on each end of the optical fibers (2, 3), and a metal ring (21) placed on the circumference of the measuring end portion (6) to which an electrical contact with the object tissue is made by means of at least one lead wire (22) and one of said connecting plug of met al (20, 19) so that the degree of contact of the measuring surface of the probe (6) with object tissue (13) can be continuously observed in order to assure stable measurement of the object.

2. A probe for detecting abnormality of internal tissue by spectroscopic method utilizing optical fibers comprising:

a bundle of optical fibers (5) in which two groups of optical fibers (2, 3), namely a group of optical fibers for transmitting light beams for projection of an object tissue surface (13) and the other group of optical fibers for transmitting reflected light beams from the projected tissue surface (13) under observation;

a cover layer for protecting the bundle of fibers forming a sheath (4) over the fiber bundle (5);

a connecting portion (18) in which said two groups of optical fibers (2, 3) are divided into two independent groups of optical fibers of light-projecting and of light-receiving purposes having a connecting plug of metal (19, 20) on each end portion of said separated group of optical fibers;

characterized by further comprising

a metal ring (21) placed around the circumference of the measuring end portion (6) of said bundle of optical fibers (5), in a way that the outer diameter of the metal ring (21) does not exceed that of the bundle of optical fibers (5) covered by the protective sheath (4) by attaching the metal after removing the sheath portion lying under the metal ring off the surface of the bundle;

and at least one lead wire (22) which is bundled together with the optical fibers for the purpose of making electrical connection between the metal ring (21) on the circumference of the measuring end portion (6) of the bundle of optical fibers (5) and either one of the connecting plug of metal (19, 20) attached to the other branched end portions of the bundle.

FIG. 1

FIG. 2

FIG. 3

FIG. 4

FIG. 5

OSCILLATOR

TR

C

R

R

C

AMP

DETECTOR

FIG. 6

FIG. 7

FIG. 8